# EUROPÄISCHE PATENTSCHRIFT

(11) **EP 2 885 020 B1**
(45) Veröffentlichungstag und Bekanntmachung des Hinweises auf die Patenterteilung: **15.02.2017**
(21) Anmeldenummer: 13752589.5
(22) Anmeldetag: 16.08.2013
(51) Int. Cl.: A61M 1/36

(54) **T-STÜCK MIT TURBULENZERZEUGUNG**
T-PIECE WITH TURBULENCE GENERATION
PIÈCE EN T PRODUISANT DES TURBULENCES

(30) Priorität: 16.08.2012 DE 102012016210; 16.08.2012 US 201261683818 P
(43) Veröffentlichungstag der Anmeldung: 24.06.2015
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Ziermann, Oliver
(86) Internationale Anmeldenummer: PCT/EP2013/002482
(87) Internationale Veröffentlichungsnummer: WO 2014/026771

(56) Entgegenhaltungen:
- WO-A1-2009/030973
- US-A- 3 955 573
- US-A- 3 964 484
- US-A1- 2007 027 305
- US-A1- 2012 132 669
- "FLANGED STATIC MIXER WITH PORT SERIES 275", INTERNET CITATION, 26. Februar 2002 (2002-02-26), XP002404322, Gefunden im Internet: URL:http://www.koflo.com/images/Flanged_po rtDrawing.pdf [gefunden am 2006-10-24]

## Beschreibung

Die Erfindung betrifft ein T-Stück mit einem Mittel zur Turbulenzerzeugung.

### Stand der Technik

In der WO 2009/030973 wird ein Blutschlauchsystem mit einer Infusionsstelle beschrieben, die eine Verengung aufweist. Diese Verengung dient zur Erzeugung einer Turbulenz in dieser Infusionsstelle.

### Problemstellung

Bei extrakorporalen Blutbehandlungsmethoden wird dem Patienten Blut entnommen, dem Blut werden in einem extrakorporalen Kreislauf durch eine Behandlungsvorrichtung, wie z.B. einem Dialysefilter, schädliche Substanzen entzogen, und dann wird das behandelte Blut dem Patienten zurückgegeben. Um den Verschluss des dazu verwendeten Blutschlauchsystems und der Behandlungsvorrichtung durch Gerinnsel zu verhindern, müssen Maßnahmen zur Antikoagulation ergriffen werden.

Dazu werden hauptsächlich zwei Verfahren angewendet, die systemische und die regionale Antikoagulation. Bei der systemischen Antikoagulation wird dem Blut im Blutschlauchsystem möglichst nahe der arteriellen Punktionsnadel über ein T-Stück eine Heparinlösung zugesetzt. Bei der regionalen Antikoagulation wird als Antikoagulans eine Citratlösung verwendet, die Calcium komplexiert, und so die Gerinnungskaskade unterbricht. In dem Dialysefilter wird das Citrat zusammen mit dem daran gebundenen Calcium aus dem Blut entfernt. Mit der Rückgabe des Blutes an den Patienten muss bei diesem Verfahren die physiologische Calciumkonzentration wiederhergestellt werden, da es sonst zu Nebenwirkungen kommen kann. Deshalb wird bei der regionalen Antikoagulation dem Blut nahe dem venösen Zugang zum Patienten eine calciumhaltige Lösung zudosiert, durch die die physiologische Calciumkonzentration im Blut wiederhergestellt wird.

Um dem Blut im extrakorporalen Kreislauf Infusionslösungen zuzugeben, können im Blutschlauchsystem T-Stücke vorgesehen sein. Diese können z.B. bei der regionalen Antikoagulation an die Antikoagulationslösung und die calciumhaltige Substitutionslösung angeschlossen werden. Die Flussrate der zuzugebenden Lösungen ist dabei im Allgemeinen im Verhältnis zum Blutfluss gering (z.B. 10ml/h zu 100 ml/min).

Aufgrund des kreisförmigen Querschnitts und der glatten Innenwandung an der Zugabestelle liegen innerhalb des T-Stücks laminare Strömungsbedingungen vor. Diese weitgehend laminaren Strömungsbedingungen bedingen eine schlechte Durchmischung des Blutes mit der zugegebenen Lösung. Diese mangelhafte Vermischung der beiden Flüssigkeiten an der Zugabestelle ist speziell bei der Zugabe der Calciumlösung zu Blut unerwünscht und führt vereinzelt zu Gerinnselbildung flussabwärts.

Um dies zu verhindern ist die rasche und homogene Durchmischung der zugegebenen Calciumlösung mit dem Blut vorteilhaft.

Die Aufgabe der vorliegenden Erfindung ist die Bereitstellung eines T-Stücks, das eine rasche und homogene Durchmischung der zwei im T-Stück zusammengeführten Flüssigkeiten gewährleistet.

Nach der Lehre der vorliegenden Erfindung wird diese Aufgabe gelöst durch ein Blutschlauchsystem nach Anspruch 1. Vorteilhafte Ausgestaltungen sind Gegenstand der Unteransprüche.

### Zusammenfassung der Erfindung

Die Erfindung betrifft ein Einsatzstück, z.B. einem T-Stück, für ein Blutleitungssystem mit einer Hauptleitung zum Transport einer ersten Flüssigkeit, vorzugsweise Blut. Die Hauptleitung besteht aus einer ersten Anschlussstelle, die über ein Zwischenstück mit einer weitgehend zylinderförmigen Innenwand zu einer zweiten Anschlussstelle führt.

Weiterhin weist das Einsatzstück eine zweite Leitung zum Dosieren einer zweiten Flüssigkeit zu der ersten Flüssigkeit auf, die von einer dritten Anschlussstelle zu einer Mündung im Zwischenstück der Hauptleitung führt. Das Zwischenstück der Hauptleitung weist dabei eine dreidimensionale Spiralstruktur zur Erzeugung einer Turbulenz auf.

Diese dreidimensionale Spiralstruktur kann als erhöhter Wulst auf der Innenwand des Zwischenstücks ausgebildet sein.

Alternativ dazu kann die dreidimensionale Spiralstruktur auch als vertiefter Ausschnitt in der Innenwand des Zwischenstücks ausgeführt werden.

Die Spiralstruktur kann trapezförmig ausgebildet sein. Alternativ kann die Spiralstruktur eine sichelförmige Ausbildung aufweisen.

Die Spiralstruktur ist als eingängige Spiralstruktur ausgebildet , wobei die eingängige Spiralstruktur die Innenwand in einem Winkel kleiner 360° umläuft. Dies hat vor allem bei der Anfertigung des Spritzgusswerkzeuges zur Herstellung des Einsatzstücks Vorteile.

Alternativ dazu, aber nicht erfindungsgemäß, kann die dreidimensionale Spiralstruktur auch als mehrgängige Spiralstruktur ausgeführt sein.

In einer weiteren Ausführungsform kann das Einsatzstück auch in zwei Teilen ausgeführt sein. Dann besteht die dreidimensionale Spiralstruktur aus einem gebogenen, drahtförmigen Material, das in das Einsatzstück eingelegt oder eingeklebt sein kann.

Um eine möglichst effiziente Durchmischung zu erreichen, befindet sich in einer bevorzugten Ausführungsform die Mündung der zweiten Leitung in der stromaufwärtigen Hälfte des Zwischenstücks. Die Mündung der zweiten Leitung soll möglichst nahe an der Anschlussstelle am Eingang des Einsatzstücks liegen. Die Mündung der zweiten Leitung soll möglichst nahe an der Spiralstruktur liegen. So werden die erste und die zweite Flüssigkeit über eine möglichst lange Distanz durch die Vorrichtung zur Erzeugung einer Turbulenz geführt und besonders effizient gemischt.

In einer besonders bevorzugten Ausbildungsform ist die Einmündung neben der stromabwärts gelegenen Flanke des Wulstes angeordnet. Dann befindet sich die Einmündung nicht symmetrisch zwischen zwei Wulsten, sondern ist unsymmetrisch verlagert zu der stromabwärts gelegenen Flanke hin.

In einer bevorzugten Ausführungsform sind die Anschlussstellen mit einem Schlauchsitz ausgebildet.

Die Erfindung betrifft weiterhin ein Blutschlauchsystem zur Herstellung eines extrakorporalen Kreislaufs für ein Blutverfahren mit mindestens einem Einsatzstück. Dieses Blutschlauchsystem kann für die Durchführung einer Hämodialyse, einer Hämofiltration oder einer Hämodiafiltration geeignet sein. Das Blutschlauchsystem würde dann eine erste, arterielle Leitung zur Entnahme des Blutes umfassen, die zu einem Blutfilter führt. Der Blutfilter kann getrennt geliefert werden, kann aber auch mit dem Schlauchsystem vorkonnektiert sein. Der Blutfilter kann ein Dialysefilter, ein Filter für die Hämofiltration oder ein Filter für die Plasmapherese oder Vollblutadsorption sein. Ein Ende der ersten, arteriellen Leitung kann mit einer Entnahmekanüle versehen sein, das zweite Ende ist mit dem Blutfilter verbunden. In diesem Leitungsstück kann auch ein Einsatzstück in Form eines T-Stücks vorgesehen sein, dass zur Einleitung eines Antikoagulanzmittels in das Blut dient. Weiterhin können die üblichen Bestandteile eines Blutschlauchsystems wie eine Tropfkammer oder Einsätze für die Blutpumpen vorgesehen sein. Von dem Blutfilter aus würde dann eine zweite, venöse Leitung zum Patienten zurückführen. In einer bevorzugten Ausführungsform würde das Einsatzstück in der zum Patienten zurückführenden venösen Leitung eingesetzt sein. Die vom Blutfilter zum Patienten führende, venöse Leitung würde dann aus zwei Teilstücken bestehen. Das erste, venöse Teilstück ist auf einer Seite mit dem Blutfilter und auf der anderen Seite mit der ersten Anschlussstelle, dem Eingang des Einsatzstückes verbunden. Vorzugsweise wird der Schlauch in den Schlauchsitz des Einsatzstückes eingeklebt. Das zweite, venöse Leitungsstück würde dann mit der zweiten Anschlussstelle, dem Ausgang des Einsatzstücks verbunden sein. Auch dieses wird vorzugsweise in den Schlauchsitz des Einsatzstückes eingeklebt. Die dritte Anschlussstelle des Einsatzstücks ist mit einer dritten Leitung verbunden. Diese Leitung ist auf einer Seite in den Schlauchsitz eingeklebt sein. Das andere Ende der Leitung kann mit einem Verbindungsmittel für eine Infusionslösung versehen sein. Dieses Verbindungsmittel kann ein Luer-Konnektor, eine Kanüle, ein Spike oder Ähnliches sein. Die Infusionslösung dient in einer bevorzugten Ausführungsform zur Wiederherstellung der physiologischen Ionenkonzentration des Blutes. Sie kann z.B. zur Substitution des durch die regionale Antikoagulation reduzierten Calciumgehalts des Blutes dienen.

Die zweite, venöse Leitung kann weiterhin die in Blutschlauchsystemen üblichen, dem Fachmann bekannten Komponenten enthalten, wie eine Tropfkammer, weitere Einsatzstücke in Form von T-Stücken oder Zuspritzstellen usw.

Das Blutschlauchsystem ist in einer bevorzugten Ausführungsform zur Durchführung einer extrakorporalen Blutbehandlung mit einer regionalen Antikoagulation geeignet.

Des Weiteren betrifft die Erfindung eine Anordnung mit einem Einsatzstück, wobei die erste und die zweite Anschlussstelle mit einer blutführenden Leitung des Blutschlauchsystems verbunden ist und die dritte Anschlussstelle mit einer Leitung zu einer physiologischen Elektrolytlösung verbunden ist. In einer bevorzugten Ausführungsform handelt es sich bei der physiologischen Elektrolytlösung um eine Lösung zur Wiederherstellung der physiologischen Calciumkonzentration. Damit handelt es sich bei der Anordnung um ein Behandlungsset für die regionale Antikoagulation.

Weitere Einzelheiten und Vorteile der Erfindung werden anhand der in den Zeichnungen dargestellten Ausführungsbeispiele näher beschrieben. Sie zeigen:
Figur 1: Schematische Darstellung eines extrakorporalen Kreislaufs mit regionaler Antikoagulation
Figur 2: Längsschnitt eines Einsatzstücks mit dreidimensionaler Spiralstruktur einer ersten Ausführungsform eines erfindungsgemäßen Blutschlauchsystems.
Figur 3: Perspektivische Darstellung eines Einsatzstücks mit dreigängiger Spiralstruktur eines nicht erfindungsgemäßen Blutschlauchsystems.
Figur 4: Querschnitt eines Einsatzstücks eines erfindungsgemäßen Blutschlauchsystems.
Figur 5: Längsschnitt eines Einsatzstücks mit stromaufwärtiger Position der dritten Anschlussstelle eines erfindungsgemäßen Blutschlauchsystems.

### Beschreibung

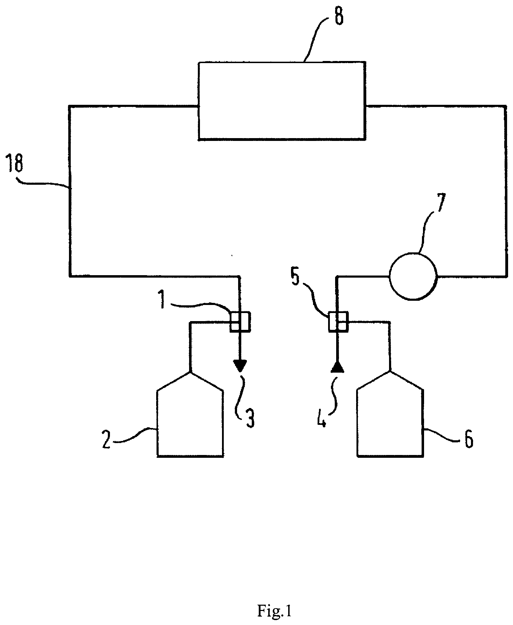

Figur 1 zeigt schematisch einen extrakorporalen Kreislauf mit regionaler Antikoagulation. Von dem arteriellen Patientenzugang 4 wird Blut von der Blutpumpe 7 in das Blutschlauchsystem 18 des extrakorporalen Kreislaufs gepumpt. Möglichst nahe am arteriellen Patientenzugang 4 befindet sich ein T-Stück 5, in dem das Blut mit einer CitratLösung 6 antikoaguliert wird. Danach passiert das Blut dann den Dialysefilter 8, in dem Citrat und zu einem Teil auch komplexiertes Calcium entfernt werden. Vor Rückgabe an den Patienten über den venösen Patientenzugang 3 wird das Blut in einem T-Stück 1 mit einer Calcium-Lösung 2 vermischt, so dass sich eine physiologische Blutzusammensetzung ergibt.

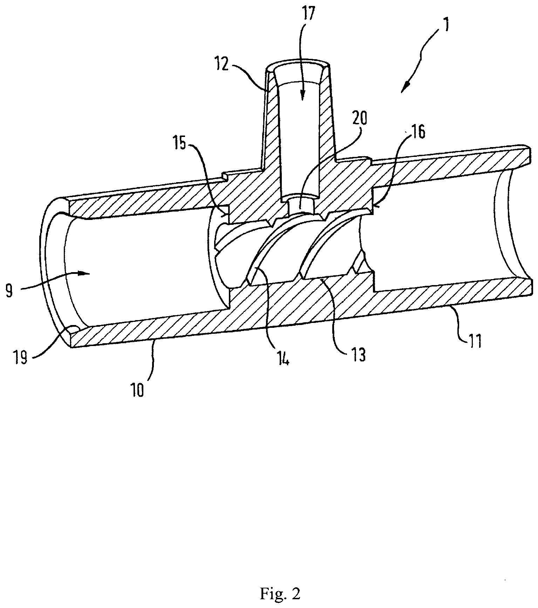

In Figur 2 wird der Längsschnitt eines Einsatzstücks 1 in Form eines T-Stücks gezeigt. Das Einsatzstück 1 weist eine Hauptleitung 9 auf, die von einer ersten Anschlussstelle 10 über ein Zwischenstück 13 mit einer zylinderförmigen Innenwand zu einer zweiten Anschlussstelle 11 führt. Die Übergangsstellen zwischen Anschlussstellen 10 und 11 und dem Zwischenstück 13 bilden jeweils einen Schlauchsitz 15 und 16 für das Blutschlauchsystem 18 (nicht gezeigt). Zur Vereinfachung des Einsetzens des Blutschlauchsystems in die Anschlussstellen 10 und 11 weisen diese eine Einführschräge 19 auf. Weiterhin weist das Einsatzstück 1 eine zweite Leitung 17 auf, die von einer dritten Anschlussstelle 12 zu einer Mündung 20 im Zwischenstück 13 in der Hauptleitung 9 führt. Das Zwischenstück 13 weist eine dreidimensionale Spiralstruktur 14 in Form eines erhöhten Wulstes auf der Innenwand auf. Diese Spiralstruktur führt zu einer Erzeugung einer Turbulenz in der in der Hauptleitung geförderten Flüssigkeit, z.B. in Blut. Die in einer zweiten Leitung 17 geförderte Flüssigkeit, z.B. eine Calcium-Lösung, die über die dritte Anschlussstelle 12 mit dem Zwischenstück der Hauptleitung verbunden ist, wird dadurch schneller mit der Flüssigkeit in der Hauptleitung 9 durchmischt.

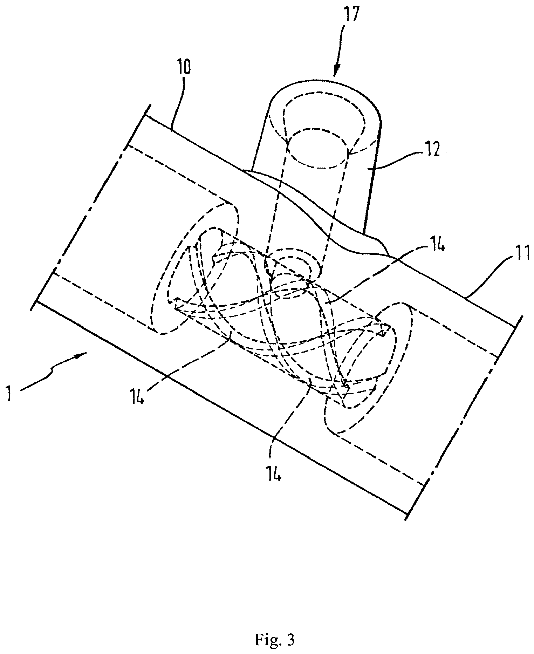

In Figur 3 ist ein Einsatzstück 1 mit einer mehrgängigen Spiralstruktur eines nicht erfindungsgemäßen Blutschlauchsystems gezeigt. Das Zwischenstück 13 weist dabei an der Innenwand drei spiralförmig verlaufende, erhöhte Wulste 14 auf.

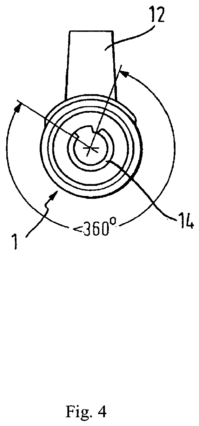

Figur 4 zeigt einen Querschnitt durch ein Einsatzstück 1 eines erfindungsgemäßen Blutschlauchsystems. Der Umschlingungswinkel des spiralförmig verlaufenden Wulstes 14 beträgt weniger als 360°. So ist das Einsatzstück spritzgusstechnisch einfach herzustellen und trotzdem wird eine ausreichende Turbulenz erzeugt.

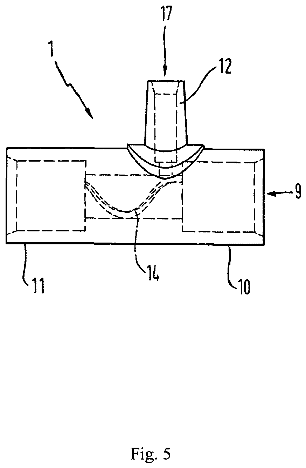

In Figur 5 ist ein Längsschnitt durch das Einsatzstück 1 gezeigt.

Die Mündung 20 ist dabei stromaufwärtig in dem Zwischenstück 13 möglichst nahe an der Spiralstruktur 14 angeordnet. Das Blut in der Hauptleitung 9 wird so optimal mit der Calciumlösung, die über die zweite Leitung 17 zudosiert wird, durchmischt. Ferner befindet sich die Einmündung der Leitung 17 neben der stromabwärts gelegenen Flanke des Wulstes 14, wegen günstiger Turbulenz- und Druckverhältnisse.

Bei extrakorporalen Kreisläufen muss neben der möglichen Gerinnung auch die Blutverträglichkeit beachtet werden. Im Blut sind empfindliche Zellen für die auch die mechanischen Belastungen im Kreislauf möglichst gering gehalten werden müssen. An Verengungsstellen im Blutschlauchsystem ist das Blut erhöhten Scherkräften ausgesetzt, die möglichst vermieden werden sollen.

In dem erfindungsgemäßen T-Stück ist der Durchmesser der Innenwand des Zwischenstücks so angelegt, dass er dem Durchmesser des Blutschlauchsystems entspricht. In dem T-Stück mit der Turbulenzerzeugung durch die Spiralstruktur ist das Blut kaum Scherkräften ausgesetzt. Die erfindungsgemäße Vorrichtung ist damit besonders hämokompatibel.

## Patentansprüche

1. Blutschlauchsystem (18) zur Herstellung eines extrakorporalen Kreislaufs für die Durchführung einer Hämodialyse, einer Hämofiltration oder einer Hämodiafiltration oder einer Plasmapheresebehandlung mit einem Einsatzstück (1),umfassend eine Hauptleitung (9) zum Transport einer ersten Flüssigkeit, vorzugsweise Blut,
die von einer ersten Anschlussstelle (10) über ein Zwischenstück (13) mit einer weitgehend zylinderförmigen Innenwand zu einer zweiten Anschlussstelle (11) führt,
und umfassend eine zweite Leitung (17) zum Dosieren einer zweiten Flüssigkeit zu der ersten Flüssigkeit, die von einer dritten Anschlussstelle (12) zu einer Mündung (20) im Zwischenstück (13) der Hauptleitung (9) führt,
**dadurch gekennzeichnet, dass** das Zwischenstück (13) der Hauptleitung (9) eine dreidimensionale, eingängige Spiralstruktur (14) zur Erzeugung einer Turbulenz aufweist, die die Innenwand in einem Winkel < 360° umläuft.

2. Blutschlauchsystem (18) nach Anspruch 1 **dadurch gekennzeichnet, dass** die dreidimensionale Spiralstruktur(14) als erhöhter Wulst auf der Innenwand des Zwischenstücks (13) ausgebildet ist.

3. Blutschlauchsystem (18) nach Anspruch 1 **dadurch gekennzeichnet, dass** die dreidimensionale Spiralstruktur (14) als vertiefter Ausschnitt in der Innenwand des Zwischenstücks ausgebildet ist.

4. Blutschlauchsystem (18) nach Anspruch 2 oder 3 **dadurch gekennzeichnet, dass** die dreidimensionale Spiralstruktur (14) trapezförmig oder sichelförmig ausgebildet ist.

5. Blutschlauchsystem (18) nach einem der vorangehenden Ansprüche **dadurch gekennzeichnet, dass** sich die Einmündung (20) der zweiten Leitung (17) in der stromaufwärtigen Hälfte des Zwischenstücks (13) befindet.

6. Blutschlauchsystem (18) nach einem der Ansprüche 1 bis 5 **dadurch gekennzeichnet, dass** die Einmündung (20) der zweiten Leitung (17) neben der stromabwärtig gelegenen Flanke des Wulstes angeordnet ist.

7. Blutschlauchsystem (18) nach einem der Ansprüche 1 bis 6 **dadurch gekennzeichnet, dass** es für die Durchführung einer regionalen Antikoagulation geeignet ist.

8. Blutschlauchsystem (18) nach Anspruch 1 bis 7, wobei die erste (10) und die zweite Anschlussstelle (11) des Einsatzstücks (1) mit einer blutführenden Leitung des Blutschlauchsystems (18) verbunden sind und die dritte Anschlussstelle (12) mit einer Leitung zu einer Elektrolytlösung verbunden ist.

9. Blutschlauchsystem nach Anspruch 8 **dadurch gekennzeichnet, dass** die Elektrolytlösung die physiologische Calciumkonzentration des Blutes wiederherstellt.

## Claims

1. A blood tubing system (18) for establishing an extracorporeal circulation for carrying out hemodialysis, hemofiltration or hemodiafiltration or plasmapheresis treatment with an insert piece (1) comprising a main line (9) for conveying a first liquid, preferably blood,
leading from a first connection (10) to a second connection (11) via an intermediate piece (13) having a largely cylindrical inside wall,
and comprising a second line (17) for dosing a second liquid to the first liquid leading from a third connection (12) to an orifice (20) in the intermediate piece (13) of the main line (9),
**characterized in that** the intermediate piece (13) of the main line (9) has a three-dimensional single-start spiral structure (14) for creating turbulence, revolving around the inside wall at an angle <360°.

2. The blood tubing system (18) according to claim 1,
**characterized in that** the three-dimensional spiral structure (14) is designed as an elevated bead on the inside wall of the intermediate piece (13).

3. The blood tubing system (18) according to claim 1,
**characterized in that** the three-dimensional spiral structure (14) is designed as a recessed cutout in the inside wall of the intermediate piece.

4. The blood tubing system (18) according to claim 2 or 3, **characterized in that** the three-dimensional spiral structure (14) is designed to be trapezoidal or crescent-shaped.

5. The blood tubing system (18) according to any one of the preceding claims, **characterized in that** the orifice (20) in the second line (17) is located in the upstream half of the intermediate piece (13).

6. The blood tubing system (18) according to any one of claims 1 through 5, **characterized in that** the orifice (20) of the second line (17) is arranged next to the downstream flank of the bead.

7. The blood tubing system (18) according to any one of claims 1 through 6, **characterized in that** it is suitable for carrying out regional anticoagulation.

8. The blood tubing system (18) according to any one of claims 1 through 6, wherein the first connection (10) of the insert piece (1) and the second connection (11) are connected to a blood-carrying line of the blood tubing system (18), and the third connection (12) is connected to a line leading to an electrolyte solution.

9. The blood tubing system according to claim 8, **characterized in that** the electrolyte solution restores the physiological calcium concentration of blood.

## Revendications

1. Système de tuyau sanguin (18) pour l'établissement un circuit extracorporel pour la réalisation d'une hémodialyse, d'une hémofiltration ou d'une hémodiafiltration ou d'un traitement par plasmaphérèse avec un insert (1), comprenant une conduite principale (9) pour le transport d'un premier liquide, de préférence du sang,
qui va d'un premier point de raccordement (10) en passant par une pièce intermédiaire (13) comportant une paroi intérieure dans une large mesure de forme cylindrique vers un deuxième point de raccordement (11),
et comprenant une deuxième conduite (17) pour le dosage d'un deuxième liquide dans le premier liquide et qui va d'un troisième point de raccordement (12) vers une embouchure (20) de la pièce intermédiaire (13) de la conduite principale (9),
**caractérisé en ce que** la pièce intermédiaire (13) de la conduite principale (9) présente une structure spiralée tridimensionnelle à un pas (14) pour générer une turbulence qui passe autour de la paroi intérieure sur un angle < 360°.

2. Système de tuyau sanguin (18) selon la revendication 1, **caractérisé en ce que** la structure spiralée tridimensionnelle (14) est réalisée sous forme d'un bourrelet surélevé sur la paroi intérieure de la pièce intermédiaire (13).

3. Système de tuyau sanguin (18) selon la revendication 1, **caractérisé en ce que** la structure spiralée tridimensionnelle (14) est réalisée sous forme d'une découpe creusée dans la paroi intérieure de la pièce intermédiaire.

4. Système de tuyau sanguin (18) selon la revendication 2 ou 3, **caractérisé en ce que** la structure spiralée tridimensionnelle (14) est réalisée en forme de trapèze ou en forme de faucille.

5. Système de tuyau sanguin (18) selon une des revendications précédentes, **caractérisé en ce que** l'embouchure (20) de la seconde conduite (17) se trouve dans la moitié amont de la pièce intermédiaire (13).

6. Système de tuyau sanguin (18) selon une des revendications 1 à 5 **caractérisé en ce que** l'embouchure (20) de la seconde conduite (17) est disposée près du flanc situé en aval du bourrelet.

7. Système de tuyau sanguin (18) selon une des revendications 1 à 6, **caractérisé en ce qu'**il est adapté pour réaliser une anticoagulation locale.

8. Système de tuyau sanguin (18) selon la revendication 1 à 7, dans lequel les premier (10) et deuxième points de raccordement (11) de l'insert (1) sont raccordés à une conduite conductrice de sang du système de tuyau sanguin (18) et que le troisième point de raccordement (12) est raccordé à une conduite menant vers une solution électrolytique.

9. Système de tuyau sanguin selon la revendication 8, **caractérisé en ce que** la solution électrolytique rétablit la concentration physiologique en calcium du sang.
